# EUROPEAN PATENT SPECIFICATION

(11) **EP 2 834 349 B1**
(45) Date of publication and mention of the grant of the patent: **20.04.2016**
(21) Application number: 14738871.4
(22) Date of filing: 19.06.2014
(51) Int. Cl.: C12N 5/077

(54) **P38 MAPK INHIBITOR-CONTAINING MEDIUM AND METHOD FOR OSTEOGENIC DIFFERENTIATION**
MEDIUM MIT EINEM P38-MAPK-HEMMER UND VERFAHREN ZUR OSTEOGENEN DIFFERENZIERUNG
MILIEU CONTENANT UN INHIBITEUR DE LA MAPK P38 ET MÉTHODE DE DIFFÉRENCIATION OSTÉOGÉNIQUE

(30) Priority: 20.06.2013 FI 20135675
(43) Date of publication of application: 11.02.2015
(73) Proprietor: Tampereen Yliopisto, 33014 Tampereen Yliopisto (FI)
(72) Inventor: MIETTINEN, Susanna, FI-33014 Tampereen Yliopisto (FI); VANHATUPA, Sari, FI-33014 Tampereen Yliopisto (FI)
(74) Representative: Kolster Oy Ab
(86) International application number: PCT/FI2014/050497
(87) International publication number: WO 2014/202837

(56) References cited:
- WO-A2-2005/053795
- WO-A2-2007/016621
- THI KIM PHUONG DOAN ET AL: "Inhibition of JNK and ERK pathways by SP600125- and U0126-enhanced osteogenic differentiation of bone marrow stromal cells", TISSUE ENGINEERING AND REGENERATIVE MEDICINE, vol. 9, no. 6, 30 November 2012 (2012-11-30), pages 283-294, XP055136831, ISSN: 1738-2696, DOI: 10.1007/s13770-012-0352-6
- LIU Y ET AL: "Function of TGF-beta and p38 MAKP signaling pathway in osteoblast differentiation from rat adipose-derived stem cells", EUROPEAN REVIEW FOR MEDICAL AND PHARMACOLOGICAL SCIENCES, VERDUCI EDITORE, IT, vol. 17, no. 12, 1 June 2013 (2013-06-01), pages 1611-1619, XP002713718, ISSN: 1128-3602
- KARIN A. PAYNE ET AL: "Effect of Phosphatidyl Inositol 3-Kinase, Extracellular Signal-Regulated Kinases 1/2, and p38 Mitogen-Activated Protein Kinase Inhibition on Osteogenic Differentiation of Muscle-Derived Stem Cells", TISSUE ENGINEERING PART A, vol. 16, no. 12, 1 December 2010 (2010-12-01), pages 3647-3655, XP055136842, ISSN: 1937-3341, DOI: 10.1089/ten.tea.2009.0738
- STANTON ET AL: "Inhibition of p38 MAPK signaling in chondrocyte cultures results in enhanced osteogenic differentiation of perichondral cells", EXPERIMENTAL CELL RESEARCH, ACADEMIC PRESS, US, vol. 313, no. 1, 22 December 2006 (2006-12-22), pages 146-155, XP005733075, ISSN: 0014-4827, DOI: 10.1016/J.YEXCR.2006.09.027
- Y. FRIEDMANN ET AL: "JX401, A p38 Inhibitor Containing a 4-Benzylpiperidine Motif, Identified via a Novel Screening System in Yeast", MOLECULAR PHARMACOLOGY, vol. 70, no. 4, 17 July 2006 (2006-07-17), pages 1395-1405, XP055136843, ISSN: 0026-895X, DOI: 10.1124/mol.106.022962
- T ALBREKTSSON ET AL: 'Osteoinduction, osteoconduction and osseointegration' EUR SPINE J vol. 10, 30 June 2001, pages S96 - S101, XP055207255 DOI: 10.1007/ s005860100282

## Description

### TECHNICAL FIELD

The technical field concerned is treatment of bone defects and more specifically regeneration of bone tissue with mesenchymal stem cells *in vitro* and *in vivo* on subjects in need thereto. The present description is related to differentiation of adipose adult stem cells into osteoprogenitor cells and further into osteoblasts cells. Accordingly, here is provided a new culture medium comprising a supplement contributing to fast and effective differentiation of stem cells into osteogenic cells. Moreover, here is provided a cell culture component/medium supporting osteogenic potential and differentiation capacity of human adipose stem cells, cell culture and a differentiation method utilizing said new supplemented culture medium.

### BACKGROUND

Current consensus for bone tissue engineering includes three essential elements, i.e., biomaterial scaffold, osteogenic cell lineage and bone inducing factors.

Mesenchymal stem cells (MSCs) tri-lineage potential (osteogenic, chondrogenic and adipogenic lineages) have made them the focus of most experimental approaches. Research activities have been appointed to study their individual roles, as well as novel concepts on how their collective role may be the optimal strategy to improve upon *in vitro* osteogenesis and whether this could also be translated to improved bone formation *in vivo.*

Autogenous bone grafts can result in donor site morbidity and pain at the donor site, while allogenic backed bone and synthetic materials have variable effectiveness. Given these limitations, researchers have focused on new treatments that will allow for safe and successful bone repair and regeneration. MSCs have received attention for their ability to differentiate into osteoblasts, cells that synthesize the extracellular matrix and regulate matrix mineralization. Successful bone regeneration requires three elements: MSCs that serve as osteoblastic progenitors, osteoinductive growth factors and their pathways that promote development and differentiation of the cells as well as an osteoconductive scaffold that supports cell growth, differentiation and vascularization. Future treatments should strive to combine mesenchymal stem cells, cell-seeded scaffolds and osteoinductive factors to optimize the efficiency and safety of tissue repair and bone regeneration.

According to a traditional approach, for osteogenic purposes, most promising has been human bone marrow originated population of cells capable of differentiating along multiple mesenchymal cell lineages. Techniques for the purification and culture-expansion of these human marrow-derived MSCs have been developed. Reproducible systems are available for the *in vitro* osteogenic differentiation of human MSC. Osteogenic differentiation may be obtained by base media containing dexamethasone (Dex), L-ascorbic acid-2-phosphate (AsAP) or ascorbic acid, and beta-glycerophosphate (beta GP) and determined by osteoblastic morphology, expression of alkaline phosphatase (APase), reactivity with anti-osteogenic cell surface monoclonal antibodies, modulation of osteocalcin mRNA production, and the formation of a mineralized extracellular matrix containing hydroxyapatite.

The p38 MAPK inhibitor SB202190 (see below) has been used for chondrocyte cultures and resulted in enhanced osteogenic differentiation of perichondral cells (Stanton & Beier, Experimental Cell Research 313 (2007) 146-155).

### SUMMARY OF THE INVENTION

It is thus an object of the present invention to provide a medium effective to differentiate human adipose stem cells (hASCs) into osteoprogenitor cells and further into osteoblast cells. The present inventors have surprisingly found that a medium comprising a p38 MAPK inhibitor, at a concentration of 0.1 µM to less than 10 µM, contributes to the step-wise progression of cells from hASCs via undifferentiated precursors to secretory osteoblasts.

Another object of the present invention is to provide simplified medium effectively differentiating hASCs into osteoprogenitor cells and further into osteoblast cells.

A related object is to provide a medium effective to promote and guide differentiation of mesenchymal stem cells further into osteoblast cells.

Another object of the present invention is to provide a method using said medium, especially comprising a p38 MAPK inhibitor.

One object of the embodiments of present invention is to solve some problems of the prior art. The present inventors have surprisingly managed to produce osteoblast cells by differentiation of hASCs in culture medium comprising a p38 MAPK inhibitor.

Accordingly, herein is provided a differentiation medium effective to differentiate hASCs into osteoprogenitor cells and advantageously further into osteoblast cells comprising a p38 MAPK inhibitor. Preferably said cell culture medium is used for differentiation of hASCs to produce osteoblast cells or cell precursors. Hence, here is provided a method for producing osteoblast cells, osteoprogenitor cells or populations thereof, said method comprising differentiating hASCs in said differentiation medium.

Other objects, embodiments, details and advantages of the present invention will become apparent from the following figures, detailed description, examples, and dependent claims.

### BRIEF DESCRIPTION OF THE FIGURES

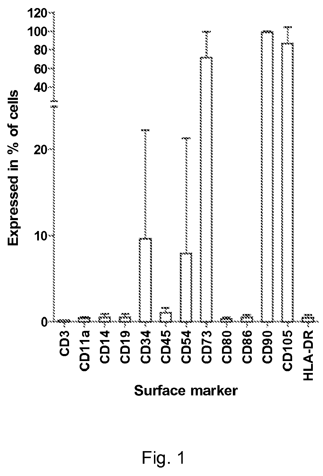

Figure 1 shows a surface marker expression profile of hASCs used in osteogenic differentiation assays.

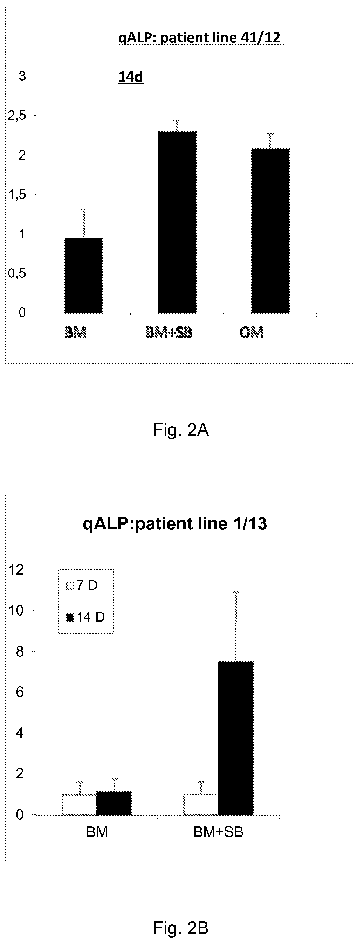

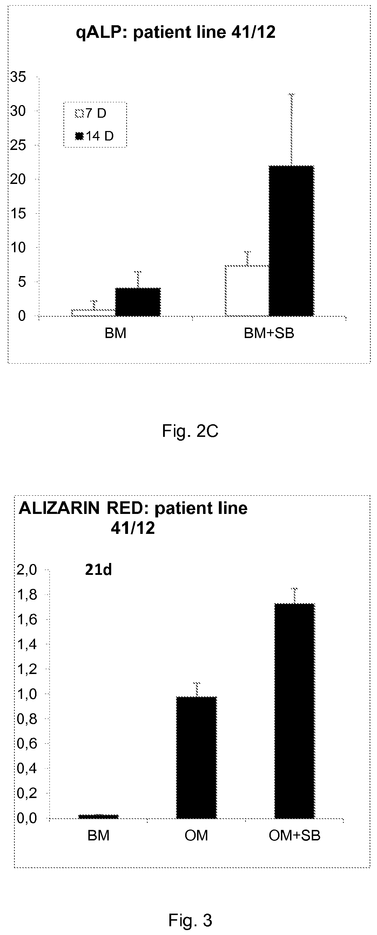

Figure 2 illustrates alkaline phosphatase (ALP) analysis in various media and for various cell lines. Results have been normalized to the amount of DNA. BM denotes basal medium, SB a p38 MAP kinase inhibitor and OM osteogenic medium. In Figure 2A these differentiation media (BM+SB and OM) are compared against BM as control. In Figure 2B and 2C two cell lines are analyzed in two time points (after 7 and 14 days) to show the applicability of the present medium to different individuals and predictability in that sense.
Figure 3 shows results from quantitative Alizarin Red/mineralization analysis of patient cell line 41/12 with and without SB inhibitor in time point 21 days. These results illustrate the potential of SB inhibitor as an enhancer of advanced processes of osteogenesis.

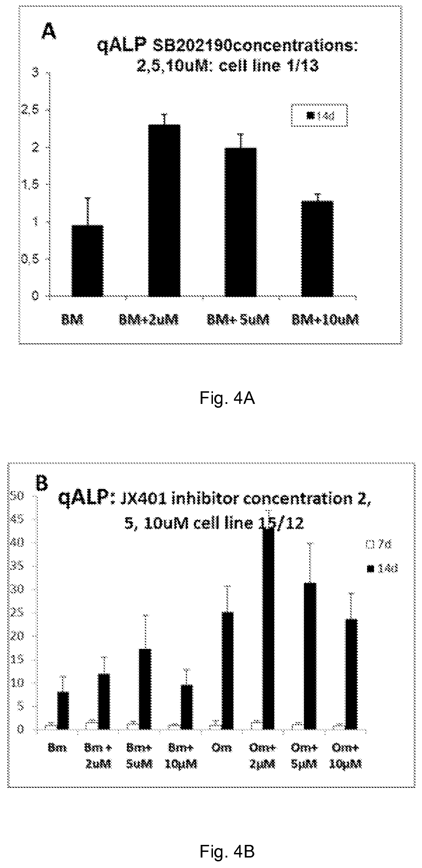

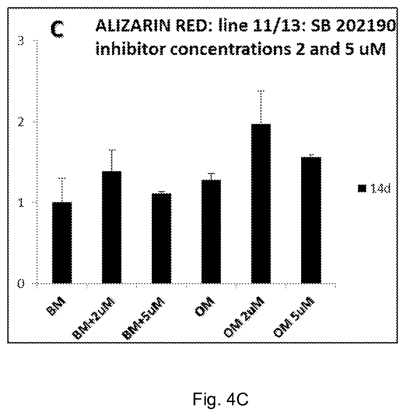

Figure 4 illustrates effective concentration ranges of p38 MAPK inhibitors on osteogenic differentiation of hASCs. In Figure 4A, the effect of SB 202190 inhibitor (2 µM, 5 µM, or 10 µM) in basic medium (BM) on quantitative alkaline phosphatase activity of hASCs (patient cell line 1/13) is shown on day 14. In Figure 4B, the effect of JX401 inhibitor (2 µM, 5µM, or 10 µM) in basic medium (BM) and osteogenic medium (OM) on quantitative alkaline phosphatase activity of hASCs (patient cell line 15/12) is shown on days 7 and 14. Figure 4C shows the effect of SB 202190 inhibitor (2 µm or 5 µM) in BM and OM on quantitative mineralization (Alizarin Red) levels of hASCs (patient cell line 11/13) on day 14. BM denotes DMEM/F12+ 5% human serum (HS, PAA Laboratories, Pasching, Austria); OM denotes DMEM/F12+ 5% HS + L-ascorbic acid 2-phosphate, β-glyserophosphate, dexamethasone.

### DETAILED DESCRIPTION OF THE INVENTION

The present description provides a medium and method for hASCs differentiation into osteogenic precursors or osteoblast cells, said medium comprising a p38 MAP kinase inhibitor. Said medium has been found to improve differentiation efficiency into osteoprogenitor cells as measured with protein marker (ALP) and/or mineralization in cell culture conditions. This differentiation medium most likely contributes to inhibition of said hASCs from adipogenic differentiation and guides them to osteogenic cells. More specifically, it controls signaling proteins involved in adipogenesis and in particular provides molecules to inhibit said signaling proteins. By this, the prerequisites for osteogenic differentiation are favored over those for adipose tissue.

The embodiments of the present invention provide several advantages. Using hASCs enables autologous cell transfers. Use of patient's own cells alleviates or removes problems related with rejecting. With hASCs ethical issues on embryonic stem cells may be avoided. Human adipose stem cells can be safely and readily isolated from adult humans in large quantities, are easy to maintain in culture, and therefore represent an ideal autologous source of cells. In humans, many cell types such as human bone marrow derived mesenchymal stem cells are not easily accessible without performing highly invasive and sometimes risky procedures. Moreover, some sources of cells are rare and not available in large quantities, making them poor candidates for reprogramming in a clinical setting.

Human ASCs have tendency to form fat cells. It has now been surprisingly shown that with medium and/or method according to the present claims, susceptibility towards differentiation into fat cells may be inhibited and hence said stem cells differentiated towards osteoblast cells.

It has now been provided a medium effective to differentiate hASCs into osteoprogenitor cells and further into osteoblast cells. The present inventors have surprisingly found that a medium comprising a p38 MAPK inhibitor contributes to the step-wise progression of cells from undifferentiated precursors to secretory osteoblasts.

### p38 MAPK and inhibition thereof

Protein kinases are involved in various cellular responses to extracellular signals. p38 Mitogen-Activated Protein (MAP) kinase (also called p38 kinase or "High Osmolarity Glycerol response kinase" (HOG)) is a member of a family of signaling molecules known as the Mitogen-Activated Protein kinase (MAP kinase or MAPK) family. Other members of the MAP kinase family include the classical MAPKs termed Extracellular signal Regulated Kinases (ERK), which are activated by a variety of mitogenic stimuli as well as differentiation signals.

p38 MAP kinase is activated by a variety of cellular stressors, including ultraviolet radiation, osmotic shock, and inflammatory cytokines, such as interleukin-1 (IL-1) and tumor necrosis factor-[alpha] (TNF-[alpha]). Once activated, p38 MAP kinase mediates the induction of mRNA synthesis for a variety of inflammatory mediators, including IL-1 [beta], TNF-[alpha], IL-6, and cyclo-oxygenase-2 (COX-2). Four isoforms of p38 MAP kinase have been identified and are designated as p38[alpha], p38[beta], p38[gamma]) and p38[delta]. p38[alpha] is also referred to as p38. p38[beta] is also referred to as p38-2. p38[gamma] is also referred to as ERK6. p38[delta] is also referred to as SAPK4. These isoforms differ in tissue expression patterns, substrate utilization, response to direct and indirect stimuli, and susceptibility to kinase inhibitors. (Yang et al, 2013). For example, it has been demonstrated that the activation of p38[beta] MAP kinase results in myocyte hypertrophy, while the activation of p38[alpha] MAP kinase leads to myocyte apoptosis. In the context of the present invention of the p38 MAPK inhibitors, p38[alpha] has shown experimentally promising results.

Inhibition of p38 MAP kinase leads to a blockade on the production of both IL-1 and TNF. IL-1 and TNF stimulate the production of other proinflammatory cytokines such as IL-6 and IL-8 and have been implicated in acute and chronic inflammatory diseases and in postmenopausal osteoporosis. Based upon this finding it is believed that p38 MAP kinase, along with other MAPKs, have a role in mediating cellular response to inflammatory stimuli, such as leukocyte accumulation, macrophage/monocyte activation, tissue resorption, fever, acute phase responses and neutrophilia. In addition, MAPKs, such as p38 MAP kinase, have been implicated in cancer, thrombin-induced platelet aggregation, immunodeficiency disorders, autoimmune diseases, cell death, allergies, osteoporosis and neurodegenerative disorders.

Molecules acting as p38 MAPK inhibitors have been widely studied and are commercially available. Publications discussing different p38 MAPK inhibitors are e.g. WO0012074 (A2), WO0026209 (A1) and EP1142890 (B1). Example of a commercially available small molecules are e.g. trans-1-(4-Hydroxycyclohexyl)-4-(4-fluorophenyl)-5-(2-methoxypyridimidin-4-yl)imidazole (SB 239063, Sigma-Aldrich), LY3007113 (Lilly) and 2,2'-Sulfonyl-bis-(3,4,6-trichlorophenol) (Calbiochem). Selecting the p38 MAPK inhibitor from small molecules provides benefits in administration. Preferably such an inhibitor is an organic molecule having a relatively low molar mass, e.g a small molecule having molar mass less than 800 g/mol, preferably less than 500 g/mol.

When selecting said p38 MAPK inhibitor from substances obtainable by chemical synthesis or recombinant production, a defined medium can be provided. Herein, the term "defined medium" refers to a composition, wherein the medium has known quantities of all ingredients. Said inhibitor also can be used in a differentiation medium that is supplemented with autologous (individuals' own) HS (autoHS). Typically the content of the p38 MAPK inhibitor in differentiation medium is between 0.1 µM and less than 10 µM, preferably between 1 µM and 8 µM, and more preferably between 2 mM and 6 µM. In some specific examples, the concentration of the p38 MAPK is 2 µM or 5 µM.

In an embodiment, the p38 MAPK inhibitor is 4-(4-Fluorophenyl)-2-(4-hydroxyphenyl)-5-(4-pyridyl)-1 H-imidazole (SB 202190) available e.g. from Sigma-Aldrich. In a preferred embodiment, the concentration of thereof is between 0.1 µM and less than 10 µM, preferably between 1 µM and 8 µM, and more preferably between 2 µM and 6 µM. In some specific examples, the concentration of SB 202190 is 2 µM or 5 µM.

In another embodiment, the p38 MAPK inhibitor is 1-[2-Methoxy-4-(methylthio)benzoyl]-4-benzylpiperidine (JX401) available e.g. from Calbiochem. In a preferred embodiment, the concentration of JX401 is between 0.1 µM and less than 10 µM, preferably between 1 µM and 8 µM, and more preferably between 2 µM and 6 µM. In some specific examples, the concentration of JX401 is 2 µM or 5 µM.

### Kit

A man skilled in the art is familiar with different culture medium concentrations. Often, culture medium is diluted and prepared to the final composition immediately before use. Therefore, it is understood that any stock solution or preparation kit suitable for use in such immediate preparation is disclosed as well. For example, for preparation of a differentiation medium according to present description, a cell differentiation kit is a common tool. Herein is disclosed a differentiation kit comprising a p38 MAPK inhibitor for preparation of a differentiation medium. It is understood that after preparation of said medium, adipose stem cells are incubated therein for a period of time and osteoprogenitor or osteoblast cells obtained. For example, for preparation of a culture medium according to present description, a cell differentiation kit comprises p38 MAPK inhibitor and optionally other components, such as basal medium or supplies for preparation thereof, as well.

### Adipose stem cells

Human ASCs (hASCs) are an attractive and abundant stem cell source with therapeutic applicability in diverse fields for the repair and regeneration of acute and chronically damaged tissues. Importantly, unlike the human bone marrow stromal/stem stem cells (BMSCs) that are present at low frequency in the bone marrow, hASCs can be retrieved in high number from either liposuction aspirates or subcutaneous adipose tissue fragments and can easily be expanded *in vitro.* ASCs display properties similar to that observed in BMSCs and, upon induction, undergo at least osteogenic, chondrogenic, adipogenic and neurogenic, differentiation *in vitro.* Furthermore, hASCs have been shown to be immunoprivileged, prevent severe graft-versus-host disease *in vitro* and *in vivo* and to be genetically stable in long-term culture. They have also proven applicability in other functions, such as providing hematopoietic support and gene transfer. Due to these characteristics, hASCs have rapidly advanced into clinical trials for treatment of a broad range of conditions (Lindroos et al., 2010).

One benefit of the present invention is that it provides a simplified medium for differentiation of adipose adult stem cells into osteoprogenitor cells and further into osteoblast cells. Simplified here refers to comparison against state of the art media, wherein several components contributing to differentiation are needed. In the state of the art osteogenic medium, the mechanism and contribution of each of the supplements to the base medium, L-ascorbic acid-2-phosphate (AsAP), β-glycerolphosphate, dexamethasone are not known and therefore are difficult to control to respond various needs and situations.

Another advantage of the present method is the effectiveness of differentiating hASCs into osteoprogenitor cells and further into osteoblast cells. Especially appreciated is the preferred differentiation into osteogenic cells over adipogenic cells.

Yet another advantage is that the present differentiation composition provides an alternative to the state of the art osteogenic medium, which contains several components the differentiation mechanism of which is not entirely known.

According to an embodiment of the invention, a p38MAPK inhibitor is provided together with osteoinductive biomaterial, which releases inhibitor molecules as it decomposes *in vivo.* Herein, an osteoinductive biomaterial refers to any biocompatible material that is able to induce osteogenesis, i.e. bone formation, of ASCs.

### Differentiation medium

Media used for cell culture have an important impact on growth and differentiation of ASCs. In the context of the present invention, hASCs may be plated and expanded in classical culture media containing balanced salt solutions such as Minimum Essential Medium MEM, Dulbecco's modified Eagle's *medium,* DMEM, medium developed by Moore et al., at Roswell Park Memorial Institute, αMEM, and DMEM:F-12 (Nutrient Mixture F-12) supplemented with either Fetal bovine serum (FBS) or human serum (HS).

In some embodiments, the medium may be supplemented with serum. From a cell culturing point of view, serum supplementation is advantageous because it provides the cells with vital nutrients, attachment factors and growth factors. Nevertheless, species of origin and serum concentrations affect the proliferation of ASCs. FBS, for instance, is known to be rich in growth factors and stimulates protein accretion in cell cultures. However, FBS replacement with HS derivatives, such as allogeneic AB serum, thrombin-activated platelet-rich plasma and human platelet lysate support equal or higher proliferation rates and multilineage differentiation capacity of ASCs.

Hence, as an embodiment the present invention, providing an autoHS differentiation medium overcomes these problems. AutoHS eliminates the problem of introducing xenogeneic or allogeneic antibodies into the patient. However, the results in terms of proliferation rate and differentiation potential may be compromised.

Furthermore, utilizing autoHS for large-scale stem cell production for clinical applications is impeded due to limited availability and high variability in cell growth in autoHS. Serum composition is largely uncharacterized, containing variable amounts of cytokines and growth factors, such as platelet derived growth factor (PDGF), BMPs and epidermal growth factor (EGF), and showing some significant lot-to-lot variability that may affect reproducibility.

### Using the differentiation medium

Differentation medium or method find use in producing osteoprogenitor cells, osteoprogenitor cell population, osteoblast cells or osteoblast cell population. These are products which are expected to contribute to treatment and research of sceletal conditions, diseases, pathologies as well as toxicology and drug development.

### Cell culture

When exploiting the differentiation media according to the present invention, in suitable conditions known to a man skilled in the art, a cell culture is established. Accordingly, herein is provided a cell culture comprising an adipose adult stem cell and differentiation medium according to claims of the present invention. Culturing said culture according to the method described in the present invention, osteoprogenitor and/or osteoblast cells can be harvested for further use.

### Method for differentiating osteoprogenitor cells

Method for differentiating osteoprogenitor cells *in vitro* comprises culturing stem cells in a differentiation medium comprising a p38 MAPK inhibitor. The time required for differentiation is dependent on the purpose, but *in vitro* applications, 7 d and 14 d durations are advantageous. In the method, any embodiment described when discussing the differentiation medium and alternatives thereof, may be selected depending on the aims and conditions at hand.

When applying the present method, the differentiation may be followed by known indicators. The most common are proteins expressed or which may otherwise be analyzed from the cells, medium or the cell culture. Preferably differentiation and thereby presence of osteoprogenitor cells is monitored and confirmed by analyzing expression of an osteoblast marker of which most preferable is alkaline phosphatase (ALP). Positive indication may be detected when majority of said osteoprogenitor cells express an osteoblast marker. Another indication is mineralization. In presence of appropriate osteogenic conditions ASCs maturate towards osteoblasts when they start to produce calcium phosphate mineral within extracellular matrix. Mineralization can be detected by Alizarin Red staining method.

### Bone Tissue Repair

The capacity of ASCs in bone tissue repair has not been investigated widely. From prior art, it is known a case, wherein calvarial defect was treated with autologous stromal vascular fraction (SVF) cells isolated and applied in a single operative procedure in combination with milled autologous bone from the iliac crest. The SVF cells were supported in place using autologous fibrin glue, and mechanical fixation was achieved with two large, resorbable macroporous sheets acting as a soft tissue barrier. The postoperative course was uneventful and new bone formation and near complete calvarial continuity was observed three months after the reconstruction (Lendeckel et al., 2004).

In another case of prior art, the problem with harvesting autologous bone was circumvented by using composite graft consisting of autologous ASCs combined with a synthetic bone substitute material, beta tricalcium phosphate, βTCP and rhBMP-2. For this purpose, ASCs were expanded *ex vivo* and combined with βTCP and rhBMP-2 prior to clinical transplantation procedures. Furthermore, the autologous ASCs were handled in clean rooms according with current GMP regulations. The postoperative healing was uneventful, mature bone structures had developed within the construct, and further rehabilitation with dental implants was carried out (Mesimäki et al. 2009). So far, 25 patients with craniomaxillofacial defects have been treated using similar constructs. Details of the protocols related to bone regeneration and the use of biomaterial therein are more specifically disclosed in Sándor et al. 2013 and Thesleff et al. 2013, which both are incorporated as reference here.

According to an embodiment of the invention, a p38MAPK inhibitor is provided together with osteoinductive biomaterial, which releases inhibitor molecules as it decomposes *in vivo,* said released p38MAPK inhibitor contributing to differentiation of adipose stem cells into osteoprogenitor cells, osteoblast cells, osteoprogenitor cell population, osteoblast cell population or bone tissue. Accordingly, herein is provided a method for differentiating adipose adult stem cells into osteoprogenitor cells *in vivo* comprising administering p38 MAPK inhibitor to the subject in need. One embodiment is a transplant comprising an adipose adult stem cell, a p38 MAPK inhibitor and osteoinductive biomaterial. Alternatively, it may be considered that an adipose adult stem cell is not necessarily transplanted but p38 MAPK inhibitor contributes to differentiation of an adipose adult stem cells present in the site of transplant.

### Experimental

The study was conducted in accordance with the ethics committee of the Pirkanmaa Hospital District, Tampere, Finland (R03058). ASCs were isolated from subcutaneous adipose tissue samples obtained with written informed consent from 4 female donors (age, 44 ± 7 years) undergoing elective surgical procedures in the Department of Plastic Surgery, Tampere University Hospital, Tampere, Finland. Isolation of hASCs from adipose tissue samples was carried out by using a mechanical and enzymatic method. The adipose tissue was minced manually into small fragments and digested with collagenase NB 6 GMP Grade (SERVA Electrophoresis GmbH, Heidelberg, Germany) in a water bath at 37°C under shaking conditions. The digested tissue was centrifuged and filtered in sequential steps through a 100-µm pore-size filter to separate the ASCs from the surrounding tissue. Cells were expanded in T75 flasks and passaged after reaching 80% confluency. Expansion was done in DMEM/F-12 1:1 (Life Technologies, Gibco, Carlsbad, CA, USA) supplemented with 1% I-analyl-l-glutamine (GlutaMAX I; Life Technologies, Gibco), 1% antibiotics (p/s; 100 U/ml penicillin, 0.1 mg/ml streptomycin; Lonza, BioWittaker, Verviers, Belgium) and 10% HS (PAA Laboratories). ASCs were detached by using TrypLE Select (LifeTechnologies, Gibco). Cell surface marker profile (Figure 1) was analyzed using flow cytometry (FACSAria; BD Biosciences, Erembodegem, Belgium) as described in Patrikoski et al (2013).

The effect obtainable with the present medium and method was studied experimentally. The differentiation into osteogenic cells was analysed following alkaline phosphatase (ALP) as early osteogenic marker and mineralization as a marker for advanced osteogenic process. Figure 2 illustrates protein expression of alkaline phosphatase (ALP) analysis in various media. Results have been normalized to the amount of DNA. BM denotes basal medium, SB a p38 MAP kinase inhibitor SB 202190 and OM an osteogenic medium. In Figure 2A these differentiation media results (BM+SB and OM) were compared against BM as a control. In Figure 2B and 2C results from two cell lines from different patients were analyzed in two time points (after 7 and 14 days) to show the independence on the source (patient-lines) of the present medium and inter-patient predictability in that sense. In these experiments, SB was used in a concentration of 2 µM.

The results indicated that p38 MAPK protein has a decisive impact on preventing osteogenic differentiation. On the other hand, inhibiting this protein with an inhibitor molecule, here SB202190, led to osteogenic differentiation in basal medium in almost equal level as with use of osteogenic medium. Very important finding was that alkaline phosphatase marker, and hence the differentiation could be detected in every patient cell line.

Figure 3 represents quantitated Alizarin Red mineralization analysis of ASCs in basal medium, osteogenic medium, with and without SB inhibitor (2 µM) in time point 21 days.

In further experiments, the effect of a concentration gradient of two different p38 MAPK inhibitors, i.e. SB 202190 and JX401, on osteogenic differentiation of hASCs was analysed. First, the effect of SB202190 inhibitor in a concentration range of 2 to 10 µM on hASCs was studied in BM culture conditions. The results indicated that the concentrations 2 and 5 µM clearly enhanced the ALP activity of the cells. However, at 10 µM, the ALP activity was diminished compared to the other two concentrations (Fig. 4A). To confirm these results, the effect of another p38 MAPK inhibitor, JX401, on ALP activity of hASCs was analysed. In this case, the analysis was performed both in BM and osteogenic medium (OM). In BM, the functional concentration peak was at 5 µM but also 2 µM was effective as compared to the highest concentration, 10 µM. However, in OM the most effective concentration of JX401 inhibitor was 2 µM (Fig. 4B).

To analyze how p38MAPK inhibitors affect the progression of osteogenic differentiation, Alizarin Red mineralization assay was performed both in BM and OM conditions. In both culture conditions, SB202190 inhibitor peaked at the concentration of 2 µM, but in OM also 5 µM was functional (Fig. 4C).

Taken together, these results indicate that concentrations lower than 10 µM, preferably 0.1 to 5 µM, is the functional concentration range of p38MAPK inhibitors to induce osteogenic differentiation of hASCs both in BM and OM conditions.

In the above experiments, the basal medium used was DMEM/F12+ 5% HSPaa serum. Said p38 MAP kinase inhibitor SB 202190 was from Sigma-Aldrich, while JX401 was from Calbiochem. Osteogenic medium representing the state of the art, was a base medium supplemented with L-ascorbic acid-2-phosphate (AsAP), β-glycerolphosphate, and dexamethasone.

The underlying mechanism is not clear to the present inventors. However, as most probable explanation, the differentiation is believed to be effected via p38MAPK inhibition and guidance away from fat cells thereof. The experimental results support this theory as similar experiment with bone marrow derived stem cells did not exhibit similar effect in differentiation (results not shown).

### References

Lendeckel, S., Jodicke, A., Christophis, P., et al. (2004). Autologous stem cells (adipose) and fibrin glue used to treat widespread traumatic calvari-al defects: case report. Journal of Craniomaxillofacial Surgery, 32, 370-373.

Lindroos, B., Suuronen, R., Miettinen, S., (2010). The Potential Adipose Stem Cells in Regenerative Medicine. Stem. Cell Rev and Per, Humana Press, DOI 10.1007/s12015-010-9193-7.

Mesimäki, K., Lindroos, B., Törnwall, J., Mauno, J., Lindqvist, C., Kontio, R., Miettinen, S., Suuronen, R. Novel maxillary reconstruction with ectopic bone formation by GMP adipose stem cells. Int J Oral Maxillofac Surg. 2009 Mar;38(3):201-9.

Sándor, G.K., Tuovinen, V.J., Wolff, J., Patrikoski, M., Jokinen, J., Nieminen, E., Mannerström, B., Lappalainen, O.-P., Seppänen, R., Miettinen, S. Adipose Stem Cell Tissue-Engineered Construct Used to Treat Large Anterior Mandibular Defect: A Case Report and Review of the Clinical Application of Good Manufacturing Practice -Level Adipose Stem Cells for Bone Regeneration. J Oral Maxillofac Surg. 2013 May;71(5):938-50.

Thesleff, T., Lehtimäki, K., Niskakangas, T., Mannerström, B., Miettinen, S. Suuronen, R., Ohman, J. Cranioplasty with adipose-derived stem cells and biomaterial. A novel method for cranial reconstruction. Neurosurgery. 2011 Jun;68(6):1535-40.

## Claims

1. An osteoinductive differentiation medium for differentiation of human adipose stem cells (hASCs) into osteoprogenitor cells, the medium comprising a p38 MAPK inhibitor at a concentration of 0.1 to less than 10 µM, wherein the p38 MAPK inhibitor is
4-(4-Fluorophenyl)-2-(4-hydroxyphenyl)-5-(4-pyridyl)-1 H-imidazole or
1-[2-Methoxy-4-(methylthio)-benzoyl]-4-benzylpiperidine.

2. The osteoinductive differentiation medium according to claim 1, wherein the concentration of the p38 MAPK inhibitor is between 2 and 6 µM.

3. The osteoinductive differentiation medium according to claim 1, wherein the concentration of the p38 MAPK inhibitor is 2 µM or 5 µM.

4. The osteoinductive differentiation medium according to any one of the preceding claims consisting of substantially autologous human serum (autoHS) and/or defined components.

5. A cell culture comprising hASCs and differentiation medium according to any one of claims 1-4.

6. A method for differentiating osteoprogenitor cells *in vitro* comprising culturing hASCs in a differentiation medium according to any one of claims 1-4.

7. The method according to claim 6 wherein majority of said osteoprogenitor cells express an osteoblast marker.

8. Method according to claim 7, wherein said osteoblast marker is alkaline phosphatase (ALP).

9. Method according to claim 7 or 8, wherein majority of said osteoprogenitor cells exhibit positive result in alizarin red test.

10. Use of osteoinductive differentiation medium according to any of claims 1-4 or method according to any one of claims 6-9 for producing osteoprogenitor cells, osteoprogenitor cell population, osteoblast cells or osteoblast cell population.

11. A transplant isolated comprising a p38 MAPK inhibitor 4-(4-Fluorophenyl)-2-(4-hydroxyphenyl)-5-(4-pyridyl)-1 H-imidazole or 1-[2-Methoxy -4-(methylthio)-benzoyl]-4-benzylpiperidine at a concentration of 0.1 to less than 10 µM; osteoinductive biomaterial; and optionally an adipose adult stem cell.

## Patentansprüche

1. Osteoinduktives Differenzierungsmedium zur Differenzierung von humanen Fettstammzellen (human adipose stem cells, hASCs) zu Osteoprogenitorzellen, wobei das Medium einen p38 MAPK-Inhibitor in einer Konzentration von 0,1 bis weniger als 10 µM umfasst, wobei der p38 MAPK-Inhibitor
4-(4-Fluorphenyl)-2-(4-hydroxyphenyl)-5-(4-pyridyl)-1 H-imidazol
oder
1-[2-Methoxy-4-(methylthio)-benzoyl]-4-benzylpiperidin
ist.

2. Osteoinduktives Differenzierungsmedium nach Anspruch 1, wobei die Konzentration des p38 MAPK-Inhibitors zwischen 2 und 6 µM beträgt.

3. Osteoinduktives Differenzierungsmedium nach Anspruch 1, wobei die Konzentration des p38 MAPK-Inhibitors 2 µM oder 5 µM beträgt.

4. Osteoinduktives Differenzierungsmedium nach einem der vorangehenden Ansprüche, bestehend aus im Wesentlichen autologem Humanserum (autoHS) und/oder definierten Komponenten.

5. Zellkultur umfassend hASCs und Differenzierungsmedium nach einem der Ansprüche 1-4.

6. Verfahren zum Differenzieren von Osteoprogenitorzellen *in vitro,* umfassend das Kultivieren von hASCs in einem Differenzierungsmedium nach einem der Ansprüche 1-4.

7. Verfahren nach Anspruch 6, wobei die Mehrheit der Osteoprogenitorzellen einen Osteoblastenmarker exprimiert.

8. Verfahren nach Anspruch 7, wobei der Osteoblastenmarker alkalische Phosphatase (ALP) ist.

9. Verfahren nach Anspruch 7 oder 8, wobei die Mehrheit der Osteoprogenitorzellen ein positives Ergebnis in einem Alizarinrot-Test aufweist.

10. Verwendung des osteoinduktiven Differenzierungsmediums nach einem der Ansprüche 1-4 oder Verfahren nach einem der Ansprüche 6-9 zum Herstellen von Osteoprogenitorzellen, einer Osteoprogenitorzellpopulation, Osteoblastenzellen oder einer Osteoblastenzellpopulation.

11. Isoliertes Transplantat, umfassend einen p38 MAPK-Inhibitor 4-(4-Fluorphenyl)-2-(4hydroxyphenyl)-5-(4-pyridyl)-1H-imidazol oder 1-[2-Methoxy-4-(methylthio)-benzoyl]-4benzylpiperidin in einer Konzentration von 0,1 bis weniger als 10 µM; osteoinduktives Biomaterial; und gegebenenfalls eine adulte Fettstammzelle.

## Revendications

1. Un milieu de différenciation ostéoinductive pour la différenciation de cellules souches adipeuses humaines (human adipose stem cells, hASCs) en cellules ostéoprogénitrices, le milieu comprenant un inhibiteur de la MAPK p38 à une concentration de 0,1 à moins de 10 µM, dans lequel l'inhibiteur de la MAPK p38 est
4-(4-fluorophényle)-2-(4-hydroxyphényle)-5-(4-pyridyle)-1H-imidazole
ou
1-[2-méthoxy-4(méthylthio)-benzoyle]-4-benzyl-pipéridine.

2. Le milieu de différenciation ostéoinductive selon la revendication 1, dans lequel la concentration de l'inhibiteur de la MAPK p38 est comprise entre 2 et 6 µM.

3. Le milieu de différenciation ostéoinductive selon la revendication 1, dans lequel la concentration de l'inhibiteur de la MAPK p38 est de 2 µM ou 5 µM.

4. Le milieu de différenciation ostéoinductive selon l'une quelconque des revendications précédentes se composant d'un sérum humain substantiellement autologue (autoHS) et/ou de composants définis.

5. Une culture cellulaire comprenant des hASCs et un milieu de différenciation selon l'une quelconque des revendications 1-4.

6. Un procédé pour différenciation de cellules ostéoprogénitrices *in vitro* comprenant la culture de hASCs dans un milieu de différenciation selon l'une quelconque des revendications 1-4.

7. Le procédé selon la revendication 6 dans lequel la majorité desdites cellules ostéoprogénitrices exprime un marqueur d'ostéoblastes.

8. Le procédé selon la revendication 7, dans lequel ledit marqueur d'ostéoblastes est la phosphatase alcaline (ALP).

9. Le procédé selon la revendication 7 ou 8, dans lequel la majorité desdites cellules ostéoprogénitrices présente un résultat positif au test à l'alizarine rouge.

10. Utilisation de milieu de différenciation ostéoinductive selon l'une quelconque des revendications 1-4 ou procédé selon l'une quelconque des revendications 6-9 pour produire des cellules ostéoprogénitrices, une population de cellules ostéoprogénitrices, des cellules d'ostéoblastes ou une population de cellules d'ostéoblastes.

11. Un greffon isolé comprenant un inhibiteur de la MAPK p38 4-(4-Fluorophényle)-2-(4-hydroxyphényle)-5-(4-pyridyle)-1H-imidazole ou 1-[2-Méthoxy-4(méthylthio)-benzoyle]-4-benzylpiperidine à une concentration de 0,1 à moins de 10 µM ; un biomatériau ostéoinductif ; et substantiellement une cellule souche adulte adipeuse.
